# EUROPEAN PATENT SPECIFICATION

(11) **EP 1 229 327 B1**
(45) Date of publication and mention of the grant of the patent: **21.12.2005**
(21) Application number: 01123495.2
(22) Date of filing: 28.09.2001
(51) Int. Cl.: G01N 33/42

(54) **Device and method for measuring the noise and porosity of asphalts**
Verfahren und Vorrichtung zur Messung des Geräuschpegels und der Porosität von Asphalt
Dispositif et procédé de mesure de bruit et de porosite des asphaltes

(30) Priority: 01.02.2001 IT MI010183
(43) Date of publication of application: 07.08.2002
(73) Proprietor: A.P.I.C.E. S.R.L., 38100 Trento TN (IT)
(72) Inventor: Osele, Claudio, 38100 Trento (IT)
(74) Representative: Petruzziello, Aldo

(56) References cited:
- DE-A- 4 213 221
- DE-A- 4 213 222
- US-A- 5 586 028
- PATENT ABSTRACTS OF JAPAN vol. 1996, no. 09, 30 September 1996 (1996-09-30) & JP 08 136532 A (BRIDGESTONE CORP), 31 May 1996 (1996-05-31)
- P. RASMUSSEN AND S. GADE: "Tyre noise measurement on a moving vehicle" BRÜEL & KJAER, [Online] no. BO 0452-11, April 1996 (1996-04), pages 1-8, XP002260208 Denmark Retrieved from the Internet: <URL:www.bksv.com/pdf/Bo0452.pdf> [retrieved on 2003-11-03]

## Description

The present invention refers to a device and a method for measuring/monitoring the noise level and porosity of road asphalts, particularly suitable for use in monitoring the condition of the asphalt forming the wearing course of roads, motorways and the like.

The noise given by asphalt is currently measured by various methods.

A system is known which uses microphones placed at a pre-established distance from the motorway carriageway to measure the noise produced by traffic. Said system has the drawback of measuring the noise at that particular time, in that place, with that traffic and is not able to give any indication as to how much the type of asphalt affects the measurement. With this system mainly the noise of the lane nearest to the microphone is measured and the noise of the other lanes cannot be determined. The measurement test is not reproducible in that the "environmental" conditions are not reproducible.

Another known system provides microphones placed in front of and/or at the side of and/or behind a wheel of a soundproofed trolley to measure the rolling noise of the wheel. Said system exploits one-wheeled trolleys, not foreseen by any national highway regulations, such as the Italian ones. Said one-wheeled trolleys require a suitable escort and cannot travel at high speed.

Another per se known system provides a trolley with loudspeakers which give out an acoustic signal of known frequency toward the asphalt and microphones which receive said acoustic signal measuring the residual noise. Said system does not measure the noise produced, but rather how much noise the asphalt is able to absorb. Moreover the sampling and data acquisition speed by said system is very low.

DE-4213222 discloses a method for measuring road roughness by carrying out spectral analysis of the signal recorded through a microphone fixed close to a vehicle tyre.

However, the obtained information does not concern the full width of the road

Various methods are known for measuring the drainability and porosity of asphalt. Drainability and porosity are related to one another. In fact there cannot be drainability unless the asphalt has pores in which water can run.

In order to measure the drainability of an asphalt the system commonly known as a "Belgian cylinder" is used. In this system a cylinder is filled with water and placed on the asphalt. In this manner the time taken for a known amount of water to flow out of the cylinder and be dispersed in the cavities of the asphalt is measured. Of course, to carry out the measurement the lane must be closed to traffic and the response of the measurement is relative only to the site in which it was carried out.

The porosity of an asphalt is measured with the system called "height in sand". Said system uses a laser beam to measure how deep the cavities in the asphalt are. The laser beam does not measure the whole drainage layer contained in the asphalt. In fact said measurement is carried out only until the laser beam meets the first obstacle. Thus this method is not able to detect whether there are other cavities that allow drainage beneath the first cavity.

A system is also known, devised by the same applicant, which measures both the noise and the porosity of the asphalt by placing a microphone behind a wheel of a trolley which moves rapidly along the asphalt and evaluating the spectrum obtained from the acoustic signal detected by the microphone.

This measurement system, called "high performance measurement", is based on a two-wheel carriage, in which a position transducer is placed on one wheel of the trolley and a microphone is placed on the right wheel of the trolley. The data measured by the transducer and by the microphone are combined, compressed and made available on a spreadsheet to have a measure of the noise and porosity of the asphalt along the course covered by the trolley.

This system too has some drawbacks and limitations.

The measurement of noise and porosity refers to a strip of asphalt equal to the imprint of the wheel of the trolley and the wheel of the trolley generally travels at a distance of about a metre from the right-hand edge of the traffic lane. The end user uses the data obtained by the system and tends to forget the above described limitations, consequently considering the situation measured only on the right-hand side of the lane analysed as applying to the whole carriageway. Consequently, this measurement system gives rise to uncertainty about the data analysed.

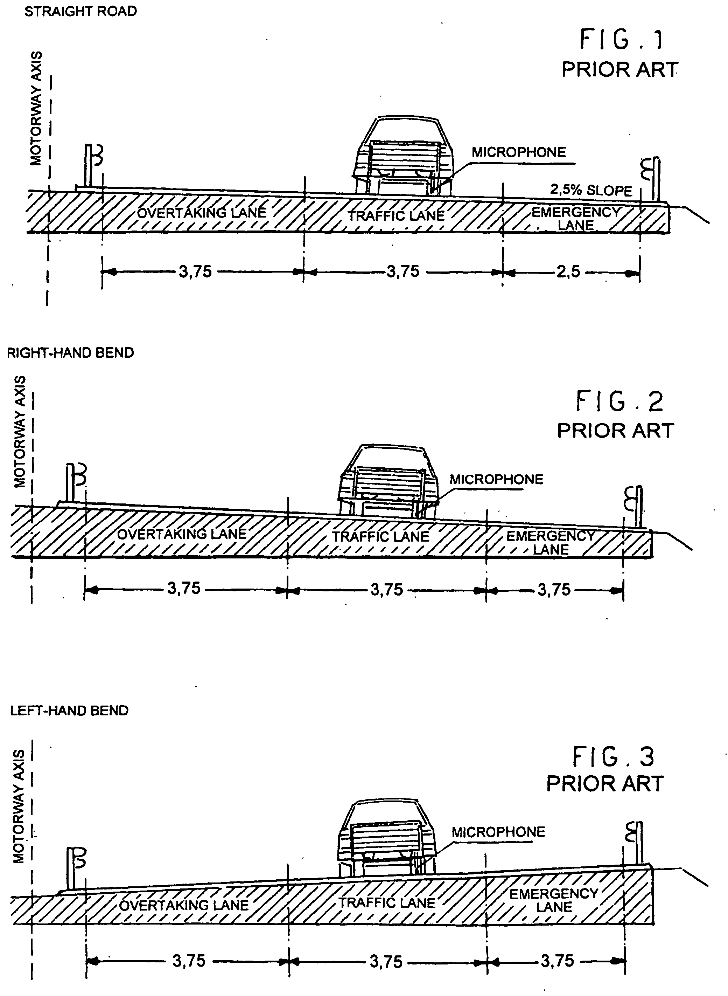

Figure 1 shows a straight stretch of a motorway roadway which has a crosswise slope, toward the right, of 2.5%. In the presence of draining asphalt, the running water carries the dirt present in the overtaking lane and in the traffic lane along the slope toward the lowest part. In some case the dirt does not flow away toward the outside but settles between the traffic lane and the hard shoulder. Since the point of measurement is about one metre from the right-hand edge of the traffic lane, the measurement is penalised by the presence of dirt. In this case the spectrum measured by the apparatus will indicate the presence of dirt and therefore a low porosity, which corresponds exactly to the situation detected.

From the data measured, the situation of the entire carriageway could be:
- dirty on the right and clean on the left for the reasons explained above,
   or
- dirty on the right and also on the left because deterioration and wear of the entire pavement is taking place. In the second case the blocking of the pores detected on the right-hand side should rightly be extended to the entire carriageway.

In conclusion, considered the phenomenon variables, a single measurement point located on the right-hand of the traffic lane cannot be extended to the entire carriageway.

As shown in Figure 2, during a right-hand bend, the slope of the carriageway toward the right increases and the previous problem re-occurs. There is also the problem of articulated trucks which tend to "cut the corner" and the tracks created by their passage are concentrated on the right. Often there is also a flattening of the asphalt caused by the increased load on the inside wheels with respect to the radius of curvature of the vehicle.

The position of the wheel of the trolley in which the measuring microphone is placed is such that the spectrum detected by the microphone, in this case also, signals blocking of the pores of the asphalt, exactly in the point of measurement, but probably not coinciding with the rest of the carriageway

In this case also the situation should be analysed as indicated above. The carriageway could be dirty on the right and clean on the left for the reasons just explained, or dirty on the right and also on the left because deterioration and wear on the entire pavement is taking place and in this latter case the finding of blocking of the porosity detected on the right-hand side should rightly be extended to the entire carriageway.

From all this it can be deduced that in this case also, the data measured with a single point of measurement offer no certainty and the phenomenon of blocking of the pores may or may not affect the entire carriageway.

As shown in Figure 3, a left-hand bend has a slope toward the left. The traffic lane is higher than the overtaking lane and the measuring wheel may be in the cleanest area of the carriageway. In this case the spectrum measured indicates the presence of open pores in the asphalt. This measurement, however, cannot be extended to the rest of the carriageway. In fact there could be problems of closure of the pores in the overtaking lane, which also extend to the traffic lane, but since measurement takes place only on the far right of the traffic lane, the phenomenon cannot be detected.

The object of the present invention is to eliminate the drawbacks of the prior art by providing a device for measuring the noise and porosity of asphalt that is efficient, versatile, practical and simple to make.

This object is achieved, according to the invention, with the characteristics listed in the appended independent claim 1.

Another object of the invention is to provide a method of measuring the noise and porosity of asphalt that is precise and reliable.

This object is achieved, in accordance with the invention, with the characteristics listed in appended independent claim 8.

Advantageous embodiments of the invention are apparent from the dependent claims.

The device and method for measuring the noise and porosity of asphalt, according to the invention, are based on the use of a trolley provided with two wheels, in which a position transducer is placed in one wheel and a microphone for detecting the acoustic signal coming from the noise between the wheel and the asphalt is positioned in each of the two wheels.

Said system has various advantages.

The measurement takes place at a constant speed and the maximum speed limit for the trolley is set by the highway code.

Neither temporary closure of lanes nor escorts for the trolley are necessary and the traffic can flow normally even while the test is being carried out, without affecting the result.

The advantages of the measurement system with two microphones according to the invention with respect to the measurement system with one microphone according to the prior art will now be pointed out.

In the measurement system according to the invention, the first microphone is positioned near the first measuring wheel of the trolley which travels at a distance of about a meter from the right-hand edge of the lane and the second microphone is positioned near the second measuring wheel of the trolley which travels at a distance of about one meter from the left-hand edge of the lane. The most obvious result is that the data obtained are doubled, since the two microphones detect a plurality of distinct sources of noise which are spaced apart from each other

In the case of homogeneous data, that is matching of the curves of the spectrums obtained from two sound sources, it is logical to think that the area between the two measured points will behave like the two measured points. Consequently, in this case, the area of measurement is no longer equal to the width of the tyre used, as in measurement with one microphone, but for homogeneous data it becomes equal to the distance between the extremities of the two wheels. That is to say, an area of measurement nearly ten times as wide as the area of measurement of the prior art system is obtained.

For the three situations seen above, straight road, right-hand bend and left-hand bend, there is no longer any difference in the analysis in that the measurement no longer takes place on the right-hand side, but also on the left-hand side, thus there is no longer any doubt about the interpretation of the data measured.

If both the right-hand microphone and the left-hand microphone signal the presence in the asphalt of closed pores, there is deterioration affecting the entire lane.

If both the right-hand microphone and the left-hand microphone signal the presence of open pores, there is in optimal situation affecting the entire lane.

If the right-hand microphone signals the presence of closed pores in the asphalt and the left-hand microphone signals the presence of open pores, there is a localised problem only on the right-hand side of the lane.

If the right-hand microphone signals the presence of open pores in the asphalt and the left-hand microphone signals the presence of closed pores, there is a localised problem on the left-hand side of the lane that cannot be detected with the system prior art which uses only one microphone on the right-hand wheel of the trolley.

Further characteristics of the invention will be made clearer by the detailed description that follows, referring to a purely exemplary and therefore non limiting embodiment thereof, illustrated in the appended drawings, in which:
Figure 1 is a schematic view illustrating from the rear a motor vehicle towing a trolley on which a prior art device for measuring the noise and porosity of the asphalt is mounted, the motor vehicle and the trolley are travelling on a straight stretch of motorway shown in section;
Figure 2 is a schematic view similar to Figure 1, in which the motor vehicle and the trolley are travelling on a stretch of motorway with a right-hand bend, shown in section;
Figure 3 is a schematic view similar to Figure 1, in which the motor vehicle and the trolley are travelling on a stretch of motorway with a left-hand bend shown in section;
Figure 4 is a schematic view similar to Figure 1, in which a device for measuring the noise and porosity of asphalt according to the invention is mounted on the trolley;
Figure 5 is a block diagram illustrating the data acquisition chain of the device for measuring the noise and porosity of asphalt according to the invention.

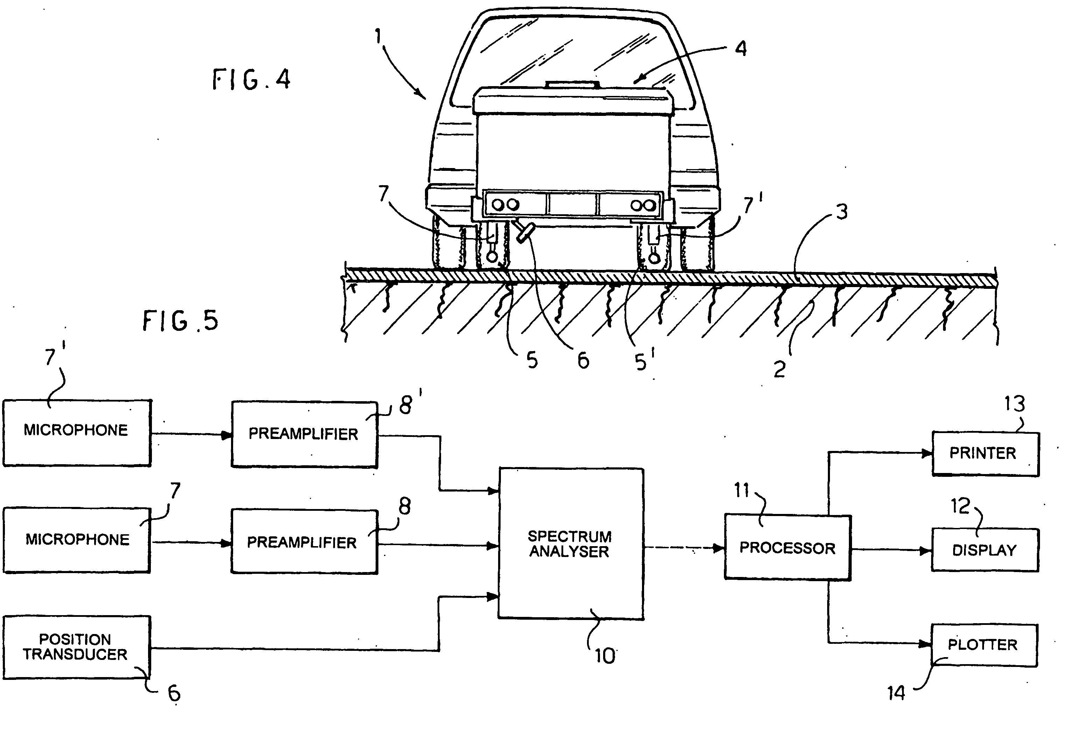

The device for measuring the noise and porosity of asphalt according to the invention will now be described with the aid of Figures 4 and 5.

Figure 4 shows a motor vehicle 1 that travels on a road surface 2 on which a layer of asphalt 3 is deposited. The motor vehicle 1 tows a trolley 4 supported by two wheels 5, 5' provided with tyres with a circumference of at least 1.75 metres and a width of 160 mm or more to have an adequate imprint on the ground. The two wheels 5, 5' mount identical tyres.

A microphone 7, 7' which has an acoustic-electric transducer is positioned behind each wheel 5, 5' of the trolley. The acoustic-electric transducer measures the acoustic pressure relating to the contact between asphalt and tyre, converting the physical phenomenon (acoustic pressure) to an analogical electronic signal (acoustic signal).

The positions of the microphones 7, 7' with respect to the source of the noise are identical. The distance of the microphones 7, 7' from the respective wheels 5, 5' is such that the measurement is not affected by the vicinity of other sources of noise.

As shown in the block diagram of Figure 5, the outputs of the analogical signal coming from the microphones 7, 7' are connected to respective signal preamplifiers 8, 8' which allow the analogical electrical signals coming from the microphones 7, 7' to be filtered and amplified.
The two signal preamplifiers 8, 8' are connected to two input channels of a multi-channel spectrum analyser 10 provided with a large storage capacity. In this manner the analogical electric signals coming from the microphones 7, 7', filtered and amplified by the signal preamplifiers 8, 8', are sent in real time to the spectrum analyser 10.

With reference to Figure 4, a position transducer 6 capable of measuring the distance covered by the trolley 4 is mounted on the left wheel 5 of the trolley. At each revolution of the wheel 5 the position transducer 6 transmits an analogical on/off signal. The position transducer 6 detects the revolutions of the wheel 5 and outputs an analogical signal in accordance with the revolutions detected.

As shown in the block diagram of Figure 5, the position transducer 6 is connected directly to an input channel of the spectrum analyser 10. In this manner, the spectrum analyser 10 records the number of revolutions made by the wheel 5 and on the basis of the circumference of the wheel 5 the distance travelled by the trolley 4 is calculated.

The data acquired by the spectrum analyser 10 during the measurement are subsequently processed in order to extract the information necessary for the final evaluation.

To allow a correct quantitative and qualitative evaluation of the sound emissions detected, the microphones 7, 7' are connected to the spectrum analyser 10 and the data measured are not recorded on recording media (DAT or other) that might alter the values transmitted.

In particular, before proceeding with combination of the data coming from the transducer 6 with the data coming from the microphones 7, 7' for determination of the values corresponding to the mileage points, some controls are performed. For this purpose the spectrum analyser 10 has additional input channels for entry of the data coming from said controls.

In particular controls are carried out on:
- instantaneous speed of coverage of the stretch of road,
- presence or absence of disturbance factors (which are signalled on the additional channels of the spectrum analyser),
- verification of the datum points for synchronization of the mileage points.

The data received by the spectrum analyser are subsequently compressed, given a geographical reference and made available in a usable format with spreadsheets.

These data are always divided by acquisition channel and thus by sound source.

The spectrum analyser has an output that can be connected to a processor. In this manner, in order to download the data measured, the spectrum analyser can occasionally be connected to a processor 11 provided with a software suitable for reading spreadsheets. The spreadsheets can be viewed by means of a display 12 connected to the processor 11 and can be printed in paper format by means of a printer 13 or plotter 14 connected to the processor 11.

Various items of information are available in a single line of the spreadsheet, the most important being:
- the mileage point to which the measurement refers;
- the acquisition channel (which identifies the position of the measuring wheel within the lane);
- the spectrum of the measurement (expressed in dBa; and present for each band of 1/3 of an octave);
- the equivalent weighted level "A" (that is, weighted according to the type "A" filter which assimilates the response of the human ear, reducing infrasounds and ultrasounds acquired by the instruments) expressed in dBa; and
- the porosity index.

The porosity index is an index for evaluating the openness or closure of the pores in the asphalt and consequently its sound deadening capacity.

A traditional bituminous carpet with closed pores has a spectrum with its maximum intensity centred between 1250 Hz and 1.600 Hz, whilst a carpet of the sound deadening draining type, thus with open pores, has a peak between 800 Hz and a maximum of 1000 Hz.

By mathematical processing of the numerical values acquired for each third of an octave, a porosity parameter is obtained to which a numerical score is given. The lower the score of the porosity parameter, the more the asphalt has open pores and therefore the more similar it is to a draining sound deadening wearing course. The higher the porosity parameter, the more the asphalt has closed pores and therefore the more similar it is to a traditional wearing course.

The present description refers specifically to a device that has two microphones associated respectively with the two wheels of a trolley. However, a trolley or other vehicle with more than two wheels can also be provided. In this case the device according to the invention will comprise a plurality of microphones associated respectively with the wheels of the trolley or of the vehicle.

Numerous variations and modifications of detail within the reach of a person skilled in the art can be made to the present embodiment of the invention, without thereby departing from the scope of the invention set forth in the appended claims.

## Claims

1. A device for measuring the noise and porosity of asphalts, **characterized in that** it comprises:
- at least two microphones (7, 7') disposed one on the right-hand side and one on the left-hand side of a vehicle, associated respectively with at least two wheels (5, 5') of the vehicle (4) so as to be able to detect the acoustic pressure caused by the contact between the tyres of the wheels (5,5') and the asphalt (3) on which the vehicle is travelling,
- a position transducer (6) able to measure the advancement of the vehicle on the asphalt (3), and
- a multi-channel spectrum analyser (10) connected to the output of the microphones (7, 7') and of the position transducer (6) to analyse the signals coming from the microphones (7, 7') and from the position transducer (6) and output data regarding the noise and porosity of the stretch of asphalt covered.

2. A device according to claim 1, **characterized in that** said vehicle is a trolley (4) with two wheels (5, 5') able to be towed by a vehicle (1).

3. A device according to claim 1 or 2, **characterized in that** interposed between said microphones (7, 7') and said spectrum analyser (10) are respective signal preamplifiers (8, 8') able to filter and amplify the signal coming from the microphones (7, 7').

4. A device according to any one of the preceding claims, **characterized in that** said spectrum analyser (10) provides additional input channels for input of the control signals obtained on:
- the instantaneous speed of coverage of the stretch of asphalt,
- the presence or otherwise of factors interfering with the acoustic pressure measurement, and
- verification of the reference data points for synchronization of the mileage points.

5. A device according to any one of the preceding claims, **characterized in that** said position transducer (6) is associated with a wheel (5) of said vehicle (4) to detect the revolutions of said wheel as it advances.

6. A device according to any one of the preceding claims, **characterized in that** said spectrum analyser (10) provides an output suitable to be connected to a processor (11) to download the data coming from said spectrum analyser (10).

7. A device according to claim 6, **characterized in that** said processor (11) is connected to a display (14) to visualize the data relating to the noise and porosity of the asphalt and a printer (13) or plotter (12) for paper printout of said data relating to the noise and porosity of the asphalt.

8. A method of measuring the noise and porosity of asphalts comprising the following steps:
- measurement of the acoustic pressure relating to the contact between asphalt and at least two tyres of a vehicle advancing on the asphalt, by means of at least two microphones, disposed one on the right-hand side and one on the left-hand side of the vehicle,
- measurement of the distance covered by the vehicle, and
- spectrum analysis of the signals indicating the acoustic pressure measured and the distance covered so as to obtain data indicating the noise and porosity of the stretch of asphalt covered.

9. A method according to claim 8, **characterized in that** a control is performed on:
- the instantaneous speed of coverage of the stretch of asphalt,
- the presence or otherwise of factors interfering with the acoustic pressure measurement, and
- verification of the datum points for synchronization of the mileage points.

## Patentansprüche

1. Eine Vorrichtung zur Messung des Lärms und der Porosität von Asphalten, **dadurch gekennzeichnet, dass** sie folgendes umfasst:
- mindestens zwei Mikrofone (7, 7'), von denen eins jeweils an der rechten und eins an der linken Seite eines Fahrzeugs angeordnet ist, welche jeweils mindestens zwei Rädern (5, 5') des Fahrzeugs (4) zugeordnet sind, um in der Lage zu sein, den akustischen Druck festzustellen, der durch den Kontakt zwischen den Reifen der Räder (5, 5') und dem Asphalt (3) verursacht wird, über den das Fahrzeug fährt.
- Ein Stellungsgeber (6), der in der Lage ist, das Vorankommen des Fahrzeugs auf dem Asphalt (3) zu messen, und
- Ein Mehrfachkanal-Spektrumsanalysator (10), der mit dem Ausgang der Mikrofone (7, 7') und des Stellungsgebers (6) verbunden ist, um die Signale zu analysieren, die jeweils von den Mikrofonen (7, 7') und dem Stellungsgeber (6) kommen, und die Ausgangsdaten, die den Lärm und die Porosität der zurückgelegten Asphalt-Teilstrecke betreffen.

2. Eine Vorrichtung gemäß Anspruch 1, **dadurch gekennzeichnet, dass** das genannte Fahrzeug ein Wagen (4) mit zwei Rädern (5, 5') ist, der von einem Fahrzeug (1) gezogen werden kann.

3. Eine Vorrichtung gemäß Anspruch 1 oder 2, **dadurch gekennzeichnet, dass** zwischen den genannten Mikrofonen (7, 7') und dem genannten Spektrumsanalysator (10) jeweils Signal-Vorverstärker (8, 8') liegen, die in der Lage sind, die Signale, die von den Mikrofonen (7, 7') kommen, zu filtern und zu verstärken.

4. Eine Vorrichtung gemäß einem beliebigen der vorausgegangenen Ansprüche, **dadurch gekennzeichnet, dass** der genannte Spektrumsanalysator (10) zusätzliche Eingangskanäle zur Eingabe der Kontrollsignale liefert, die an folgendem erzielt werden:
- der momentanen Geschwindigkeit, mit der die Asphalt-Teilstrecke zurückgelegt wird,
- dem Vorhandensein oder nicht Vorhandensein von Faktoren, die mit der akustischen Druckmessung interferieren, und
- der Prüfung der Bezugsdatenpunkte zur Synchronisation der Meilenstandspunkte.

5. Eine Vorrichtung gemäß einem beliebigen der vorausgegangenen Ansprüche, **dadurch gekennzeichnet, dass** der genannte Stellungsgeber (6) einem Rad (5) des genannten Fahrzeugs (4) zugeordnet ist, um die Umdrehungen des genannten Rads bei seinem Vorankommen festzustellen.

6. Eine Vorrichtung gemäß einem beliebigen der vorausgegangenen Ansprüche, **dadurch gekennzeichnet, dass** der genannte Spektrumsanalysator (10) einem Ausgang liefert, die geeignet ist, an einen Prozessor (11) angeschlossen zu werden, um die Daten, die von dem genannten Spektrumsanalysator (10) kommen, herunterladen zu können.

7. Eine Vorrichtung gemäß Anspruch 6, **dadurch gekennzeichnet, dass** der genannte Prozessor (11) mit einem Display (14) verbunden ist, um die Daten in Bezug auf Lärm und Porosität des Asphalts anzuzeigen, und mit einem Drucker (13) oder Aufzeichner (12) für den Ausdruck auf Papier der genannten Lärm- und Porositätsdaten des Asphalts.

8. Eine Methode zur Messung des Lärms und der Porosität von Asphalten, die folgende Schritte umfasst:
- die Messung des akustischen Drucks in Bezug auf den Kontakt zwischen Asphalt und mindestens zwei Reifen eines Fahrzeugs, das über den Asphalt fährt, mit Hilfe von mindestens zwei Mikrofonen, von denen jeweils eins an der rechten und eins an der linken Seite des Fahrzeugs angeordnet ist,
- Messung der von dem Fahrzeug zurückgelegten Strecke, und
- Spektrumsanalyse des Signals, das den akustischen Druck angibt, der gemessen wird und die zurückgelegte Entfernung, um Daten zu erhalten, die den Lärm und die Porosität der zurückgelegten Asphalt-Teilstrecke angeben.

9. Eine Methode gemäß Anspruch 8, **dadurch gekennzeichnet, dass** die Kontrolle an folgendem stattfindet:
- der momentanen Geschwindigkeit, mit der die Asphalt-Teilstrecke zurückgelegt wird,
- dem Vorhandensein oder nicht Vorhandensein von Faktoren, die mit der Messung des akustischen Drucks interferieren, und
- der Prüfung der Datenpunkte zur Synchronisation der Meilenstandspunkte.

## Revendications

1. Dispositif de mesure du bruit et de la porosité d'asphaltes, **caractérisé en ce qu'**il comprend :
- au moins deux microphones (7, 7') placés l'un sur le côté droit et l'autre sur le côté gauche d'un véhicule, associés respectivement à au moins deux roues (5, 5') du véhicule (4) de façon à être en mesure de relever la pression acoustique provoquée par le contact entre les pneumatiques des roues (5, 5') et l'asphalte (3) sur lequel le véhicule est en train de transiter,
- un transducteur de position (6) capable de mesurer l'avancement du véhicule sur l'asphalte (3), et
- un analyseur spectral multi-canal (10) relié à la sortie des microphones (7, 7') et du transducteur de position (6) pour analyser les signaux sortant des microphones (7, 7') et du transducteur de position (6) et aux données de sortie concernant le bruit et la porosité de la section d'asphalte parcourue.

2. Dispositif selon la revendication 1, **caractérisé en ce que** ledit véhicule est un chariot (4) avec deux roues (5, 5') en mesure d'être remorqué par un véhicule (1).

3. Dispositif selon la revendication 1 ou 2, **caractérisé en ce que** des préamplificateurs de signaux respectifs (8, 8') en mesure de filtrer et d'amplifier le signal sortant des microphones (7, 7') sont interposés entre les dits microphones (7, 7') et ledit analyseur spectral (10).

4. Dispositif selon une revendication quelconque parmi les revendications susmentionnées, **caractérisé en ce que** l'analyseur spectral (10) fournit des canaux d'entrée supplémentaires pour l'entrée des signaux de contrôle obtenus sur :
- la vitesse instantanée de couverture de la section d'asphalte,
- la présence ou autre de facteurs interférant avec la prise de mesure de la pression acoustique, et
- la vérification des points de données de référence pour la synchronisation des points de distance en miles.

5. Dispositif selon une revendication quelconque parmi les revendications susmentionnées, **caractérisé en ce que** ledit transducteur de position (6) est associé à une roue (5) dudit véhicule (4) pour relever les révolutions de ladite roue pendant son avancement.

6. Dispositif selon une revendication quelconque parmi les revendications susmentionnées, **caractérisé en ce que** ledit analyseur spectral (10) fournit une sortie en mesure d'être reliée à un processeur (11) pour transférer les données provenant dudit analyseur spectral (10).

7. Dispositif selon la revendication 6, **caractérisé en ce que** ledit processeur (11) est relié à un écran (14) pour afficher les données se rapportant au bruit et à la porosité de l'asphalte et une imprimante (13) ou un traceur (12) pour l'impression sur papier desdites données relatives au bruit et à la porosité de l'asphalte.

8. Méthode de mesure du bruit et de la porosité d'asphaltes comprenant les phases suivantes :
- prise de mesure de la pression acoustique se rapportant au contact entre l'asphalte et au moins deux pneumatiques d'un véhicule avançant sur l'asphalte, au moyen au moins de deux microphones, placés l'un sur le côté droit et l'autre sur le côté gauche du véhicule,
- prise de mesure de la distance parcourue par le véhicule, et
- analyse spectrale des signaux indiquant la pression acoustique mesurée et la distance parcourue de façon à obtenir des données indiquant le bruit et la porosité de la section d'asphalte parcourue.

9. Méthode selon la revendication 8, **caractérisée en ce qu'**un contrôle est réalisé sur :
- la vitesse instantanée de couverture de la section d'asphalte.
- la présence ou autre de facteurs interférant avec la prise de mesure de la pression acoustique, et
- la vérification des points de données pour la synchronisation des points de distance en miles.
